(19) **Europäisches Patentamt European Patent Office Office européen des brevets**

(11) **EP 4 617 700 A1**

(12) **EUROPEAN PATENT APPLICATION**

(43) Date of publication:
17.09.2025 Bulletin 2025/38

(21) Application number: 25163520.7

(22) Date of filing: 13.03.2025

(51) International Patent Classification (IPC):
*G01R 33/56* (2006.01)   *A61B 6/03* (2006.01)
*G06T 3/4053* (2024.01)

(52) Cooperative Patent Classification (CPC):
**G01R 33/5608; A61B 6/032; G06T 3/4053;**
G06T 2207/30061

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 14.03.2024 EP 24163586

(71) Applicant: ADIS SA
1007 Lausanne (CH)

(72) Inventors:
• WICKRAMASINGHE, Pamuditha Udaranga
1022 Chavannes-près-Renens (CH)

• BALLAN, Hussein
1806 St-Légier (CH)
• CARON, Georges
1026 Echandens (CH)
• SCHWITTER, Jürg
1095 Lutry (CH)
• HALSTENSEN, Simon
1006 Lausanne (CH)

(74) Representative: P&TS SA (AG, Ltd.)
Avenue J.-J. Rousseau 4
P.O. Box 2848
2001 Neuchâtel (CH)

(54) **A FREE-BREATHING SYSTEM AND METHOD, FOR RECONSTRUCTING A SUPER-RESOLUTION VOLUME OF A 3D PORTION OF A BREATHING BODY**

(57) The present invention concerns a free-breathing system for reconstructing a super-resolution volume (V) of a 3D portion (1) of a breathing body (100), the system comprising:
- a medical imaging device (200) comprising:
- a snapshot module arranged to move or to be moved relative to the 3D portion in a direction perpendicular to a set of K parallel snapshot planes, generating at least two snapshots for each plane within the set of K parallel planes transverse to the 3D portion, K being a non-null and positive integer number, while the body (100) is freely breathing, wherein each snapshot contains a 2D cross-section of the 3D portion

- a contour extraction module, arranged for extracting from the snapshot at least part of the contour ($C_i$) of the 2D cross-section for the snapshots ($I_i$) of each snapshot plane,
- an iterative 3D shift estimation module arranged for iteratively estimating a 3D shift in a 3D image space of the extracted contours, by using two iterative sub-steps:
- a forward step, in which the iterative 3D shift estimation module is arranged to use the extracted contours to estimate an approximate 3D shape of the 3D portion,
- a backward step, in which the iterative 3D shift estimation module is arranged to determine, for each extracted contour, the optimal 3D shift (dx, dy, dz) in the 3D image space that minimizes a discrepancy between the extracted contour and the estimated 3D shape,

- a super-resolution reconstruction module arranged for:
- repositioning in the image 3D space all snapshots according to the computed 3D shift,
- sampling the repositioned snapshots in the image 3D space with a super-resolution factor,
- computing voxel intensities in the sampled image 3D space by averaging voxel values from the snapshots, so as to reconstruct the super-resolution volume (V) of the 3D portion (1).

EP 4 617 700 A1

Fig. 1

**Description**

**Technical domain**

The present invention relates to a free-breathing system and method, for reconstructing a super-resolution volume of a 3D portion of a breathing body.

**Related art**

During a medical examination with a medical imaging device, e.g. a magnetic resonance imaging (MRI) device, a computed tomography (CT) device or any other device capable of generating volumetric images of organs, a snapshot module is arranged to generate at least two snapshots of a 3D portion of a breathing body, such as an organ or a portion of an organ of the breathing body, e.g. the heart or a portion of the heart, in a snapshot plane.

In this context, a snapshot is a 2D image. A raw, noisy image slice that MRI imaging device produces is a not limitative example of a snapshot.

In this context, a snapshot module is an image acquiring sensor, e.g. an MRI acquiring sensor. In general, there is a relative 3D motion between a snapshot module and the object or portion of the body being imaged. In this context, a snapshot module can comprise a processing unit.

For example, an MRI imaging device comprises a radio frequency (RF) coil. This RF coil is placed around the area of the body containing the organ to be examined and sends radio frequency pulses that interact with the nuclei of atoms in the body. Next, another set of RF coils detects the signals generated by these nuclei during magnetic relaxation. These signals can be then processed by a snapshot module (or by an external processor linked to the snapshot module) to generate snapshots. Snapshots are combined to create a clear slice and/or a volume that is viewed by physicians to diagnose any medical conditions.

For example, a CT imaging device includes an X-ray tube and a set of detectors. During a CT scan, the x-ray tube emits a series of thin x-ray beams through the patient's body. These X-rays are attenuated differently by body tissues, creating a three-dimensional image of internal structures. Detectors measure the attenuation of the X-rays and transmit this information to a snapshot module, which processes it to generate snapshots that are combined to create a clear slice and/or a volume that is viewed by physicians.

A motion module of the medical imaging apparatus is arranged to generate relative motion between the snapshot module and the 3D portion of the breathing body in a through-plane axis (e.g. the z-axis), which is substantially perpendicular to the snapshot plane.

In this context, the adverb "substantially" indicates that small variations from a perfect perpendicularity or parallelism can be accepted, for example, variations on the order of a few degrees.

The snapshot module is arranged to generate at least two snapshots of the 3D portion for each snapshot plane of K different snapshot planes, the K different snapshot planes being substantially parallel.

During the medical examination, the patient has two main types of movement:

- The beating of the patient's heart: this is generally not a problem, since electrodes are placed on the patient to allow synchronization of the gating with the electrocardiogram (ECG) signal;
- The patient's breathing: nowadays the patient is asked to hold his breath for at least part of the examination. Breath holding is uncomfortable for the patient and doing multiple breath holds is difficult, especially for elderly patients. In addition, it is not possible to require the patient to hold his or her breath for the entire duration of the medical examination, which generally lasts about 30 to 60 minutes.

Currently, motion correction algorithms are used to minimize motion and/or shape changes of the reconstructed organ to be examined, caused by breathing. However, these algorithms are unsatisfactory for several reasons. In general, most of them only perform in-plane motion correction, i.e., in the plane of each snapshot. As a result, significant motion artifacts are visible in the reconstructed organ when there is respiratory motion. In addition, these algorithms often have limited through-plane resolution, i.e., resolution in a direction perpendicular to the snapshot planes. The through-plane resolution is on the order of 4 mm - 6 mm, compared to an in-plane resolution of about 1 mm - 1.5 mm.

SHUZHOU JIANG ET AL., IEEE, vol 26, pages 967 - 980, ISSN 0278-0062, published on 01 July 2007 concerns MRI imaging using multi-slice snapshot images with volume reconstruction (SVR) of moving 3D objects. The method was developed for and successfully tested on moving bodies which behave as rigid bodies and so do not change size or shape, in particular fetal brains. It uses an image correlation technique which is not robust to the noise and computationally expensive. Moreover, the method relies on the computing of a fixed reference reconstructed volume. If this reference is not well computed, the method can fail.

AMEROM JOSHUA ET AL., MAGENTIC RESONANCE IN MEDICINE, ISSN 0740-3194, published on: 12 May 2019 concerns an MRI-based method using motion-corrected multi-planar imaging to obtain 4D images of the heart in real time. The method involves acquisition and reconstruction of multi-planar 2D MRI and applying an initial motion correction to obtain a rough spatial alignment using a temporal mean of the images, followed by slice-volume registration and statistical volume reconstruction. In a subsequent step, the obtained image is further refined using cardiac synchronization, in which the heart rate is first estimated independently for each slice followed by a synchronization of the of the cardiac cycle between the different imaging slices. The constant heart rate is estimated for each slice using temporal Fourier transform of the real-time image series. This method relies on an estimated heart rate to determine the position and volume of a non-rigid object. Finally, the described method relies on image correlation as well, which is not robust to the noise and computationally expensive.

**Short disclosure of the invention**

An object of the present invention is to provide a system and method for reconstructing a super-resolution volume of a 3D portion of a breathing body, which overcomes the shortcomings and limitations of the state of the art.

Another object of the invention is to provide a system and method for reconstructing a super-resolution volume of a 3D portion of a breathing body that is more robust to imaging noise (e.g. salt-and-pepper noise) and/or colour inconsistencies.

Another object of the invention is to provide a system and method for reconstructing a super-resolution volume of a 3D portion of a breathing body that is more comfortable for patients.

Another object of the invention is to provide a system and method for reconstructing a super-resolution volume of a 3D portion of a breathing body with less visible motion artifacts when there is respiratory motion and/or minor organ deformations.

Another object of the invention is to provide a system and method for reconstructing a super-resolution volume of a 3D portion of a breathing body with better through-plane and in-plane resolution.

According to the invention, these objects are achieved by the part of the attached claims, and in particular by a free-breathing system for reconstructing a super-resolution volume of a 3D portion of a breathing body according to claim 1, and by a free-breathing method for reconstructing a super-resolution volume of a 3D portion of a breathing body according to claim 15, wherein preferred embodiments of the invention are given in the dependent claims.

The free-breathing system for reconstructing a super-resolution volume of a 3D portion of a breathing body according to the invention comprises a medical imaging device comprising:

- a snapshot module arranged to move or to be moved relative to the 3D portion in a direction perpendicular to a set of K parallel snapshot planes, generating at least two snapshots for each plane within the set of K parallel planes transverse to the 3D portion, K being a non-null and positive integer number, while the body is freely breathing, wherein each snapshot contains a 2D cross-section of the 3D portion.

Advantageously, the free-breathing system according to the invention comprises also:

- a contour extraction module, arranged for extracting from the snapshot at least part of the contour of the 2D cross-section for the snapshots of each snapshot plane,
- an iterative 3D shift estimation module arranged for iteratively estimating a 3D shift in a 3D image space of the extracted contours, by using two iterative sub-steps:

  - a forward step, in which the iterative 3D shift estimation module is arranged to use the extracted contours to estimate an approximate 3D shape of the 3D portion,
  - a backward step, in which the iterative 3D shift estimation module is arranged to determine, for each extracted contour, the optimal 3D shift in the 3D image space that minimizes a discrepancy between the extracted contour and the estimated 3D shape,

- a super-resolution reconstruction module arranged for:

  - repositioning in the image 3D space all snapshots according to the computed 3D shift,
  - sampling the repositioned snapshots in the image 3D space with a super-resolution factor,
  - computing voxel intensities in the sampled image 3D space by averaging voxel values from the snapshots, so as to reconstruct the super-resolution volume of the 3D portion.

In this context, the term "contour" indicates the inner contour and/or outer contour of 3D portion in a snapshot plane, i.e. the inner and/or outer contour of the cross-section of the 3D portion as cut from a snapshot plane.

The volume of the reconstructed 3D portion has a super-resolution, since its resolution (along the through-plane axis (z) and in-plane axes (x,y)) is better than the resolution of the snapshots )along the same through-plane axis (z) and in-plane axes (x,y)). In one preferred embodiment, the super-resolution is achieved in all directions.

With respect to the known art, the invention provides the advantage that the medical examination performed by the system according to the invention is more robust to the noise, since it is based on contours. Moreover, the iterative computing performed by the iterative 3D shift estimation module allows to render it even more robust.

With respect to the known art, the invention provides the advantage that the medical examination performed by the system according to the invention is also more comfortable for the patient, since the patient can breathe freely during the entire medical examination, since a breath-hold is not required.

The invention also provides the advantage that there are less or no visible motion artifacts when there is respiratory motion.

The invention also provides the advantage that the through-plane resolution is better than the known solutions. In one embodiment, it is between 4 mm and 8 mm.

In other words, according to the invention, the patient's breathing is no longer a problem to be overcome, but rather an opportunity to be exploited to improve the resolution of the volume of the 3D portion.

In one embodiment, the system comprises also a mask computing module, arranged to compute for the snapshots of each snapshot plane a mask for an inner and/or outer contour of the 3D portion, the mask representing a segmentation of the 3D portion in each snapshot, wherein the contour extraction module is arranged for extracting from the computed masks at least part of the contour.

In one embodiment, the contour extraction module is also arranged to perform post processing on the masks, thereby generating cleaned masks. In one embodiment, the post processing comprises filtering out false positive regions from the masks, e.g. by using morphological operations.

In one embodiment, after the extraction of the masks, the contour extraction module is also arranged to place the contours back into their corresponding original (x,y,z)-locations in the image 3D space. In one embodiment, the correction is not just for z-axis, but a shift computed in step 3 is (dx,dy,dz), therefore the correction happens in-plane (x,y) and through-plane (z).

In one embodiment, a breathing pattern is used as a regularizer when determining the 3D shift.

In one embodiment, the breathing pattern is obtained from an external sensor attached to the patient, e.g. an external respiratory sensor.

In one embodiment, the breathing pattern is obtained from a motion analysis from the snapshots themselves.

In one embodiment, the system comprises also a local deformation field module, arranged to compute a local deformation field, by applying local deformations to the contours.

In one embodiment, the system comprises also a colour normalization module, arranged to perform a snapshot colour normalization of snapshots.

In one embodiment, sampling the repositioned snapshots in the image 3D space with a super-resolution factor comprises dividing the distance between a location of a snapshot and the consecutive or adjacent location by an integer number.

In one embodiment, the snapshot module is arranged for generating 24 to 32 snapshots of the 3D portion in each snapshot plane.

In one embodiment, the 3D portion of the breathing body is at least a portion of an organ, e.g. a portion of a heart.

In one embodiment, at least one module of the mentioned modules (mask computing module, contour extraction module, iterative 3D shift estimation module, local deformation field module, colour normalization module, super-resolution reconstruction module) is a machine learning-based module.

In one embodiment, the machine learning-based module is a neural network module.

In one embodiment, the 3D portion of the breathing body is at least a portion of an organ, e.g. a portion of a heart.

In one embodiment, the medical imaging device is an MRI imaging device or a CT imaging device.

The present invention concerns also a free-breathing method for reconstructing a super-resolution volume of a 3D portion of a breathing body, comprising the steps of:

- moving a snapshot module of a medical imaging device arranged relative to the 3D portion in a direction perpendicular to a set of K parallel snapshot planes,
- generating at least two snapshots for each plane within the set of K parallel planes transverse to the 3D portion, K being a non-null and positive integer number, while the body is freely breathing, wherein each snapshot contains a 2D cross-section of the 3D portion,
- extracting by a contour extraction module from the snapshot at least part of the contour of the 2D cross-section for the snapshots of each snapshot plane,
- iteratively estimating a 3D shift in a 3D image space of the extracted contours by an iterative 3D shift estimation module, by using two iterative sub-steps:

- a forward step, in which the iterative 3D shift estimation module is arranged to use the extracted contours to estimate an approximate 3D shape of the 3D portion,
- a backward step, in which the iterative 3D shift estimation module is arranged to determine, for each extracted contour, the optimal 3D shift in the 3D image space that minimizes a discrepancy between the extracted contour and the estimated 3D shape,

- repositioning by a super-resolution reconstruction module in the image 3D space all snapshots according to the computed 3D shift,
- sampling by the super-resolution reconstruction module the repositioned snapshots in the image 3D space with a super-resolution factor,
- computing voxel intensities in the sampled image 3D space by the super-resolution reconstruction module, by averaging voxel values from the snapshots, so as to reconstruct the super-resolution volume of the 3D portion.

**Short description of the drawings**

Exemplar embodiments of the invention are disclosed in the description and illustrated by the drawings in which:

Figure 1 illustrates a perspective view of a part of a medical imaging device of the free-breathing system according to an embodiment of the invention, and of a patient.

Figure 2A schematically illustrates a top view of a part of a breathing body and K different snapshot planes.

Figure 2B schematically illustrates that at each location $z_i$, N snapshots are acquired.

Figure 2C schematically illustrates a snapshot.

Figure 3A schematically illustrates top view of a part of a breathing body with the movement of the diaphragm and the heart due to the breathing, and a snapshot plane.

Figure 3B schematically illustrates the in-plane motion and the through plane motion of a point of the contour of the heart of Figure 3A, due to the breathing.

Figures 4A to 4I schematically illustrate the main steps of the free-breathing method according to an embodiment of the invention.

Figure 5A illustrates schematically a 1D object.

Figure 5B schematically illustrates the captured image of the 1D object of Figure 5A.

Figures 6A, 6C and 6E schematically illustrate a moving 1D object.

Figures 6B, 6D and 6F schematically illustrate the captured image of the 1D object of Figures 6A, 6C respectively 6E.

Figures 7A to 7F schematically illustrate the object respectively the captured images of Figures 6A to 6F after a re-alignment.

Figure 7G schematically illustrates a super-resolution image based on the aligned images of Figures 7B, 7D and 7F.

Figures 8A to 8E schematically illustrate some steps of the method according to one embodiment the invention.

Figure 9A illustrates a schematic an example of the K snapshot planes, i.e. of the snapshot planes for K locations.

Figures 9B and 9C illustrate an alternative embodiment, allowing to reduce the distance d, in order to improve the super-resolution obtained by the system according to the invention.

**Example(s) of embodiment(s) of the present invention**

In the following exemplary description, reference will be made, for simplicity's sake, to a (human) heart as a 3D portion of a breathing body. It should be understood, however, that the invention is not limited to such an organ but also includes all the organs or portions of an organ, and any 3D portion of a breathing body, as covered by the claims.

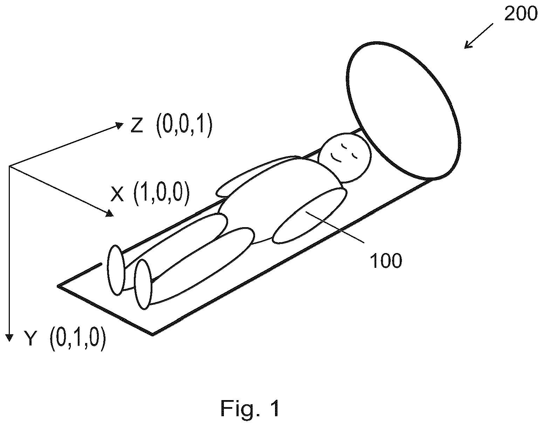

Figure 1 illustrates a perspective view of a part of a medical imaging device 200 of the free-breathing system according to an embodiment of the invention, and of a patient 100.

In one embodiment, the medical imaging device 200 has a global coordinate system X, Y, Z, wherein the vector Z defines the main direction of the patient 100, i.e. the patient's feet-to-head direction. The medical imaging device 200 can be a magnetic resonance imaging (MRI) imaging device, a computed tomography (CT) imaging device or any other device able to generate volumetric images of organs,

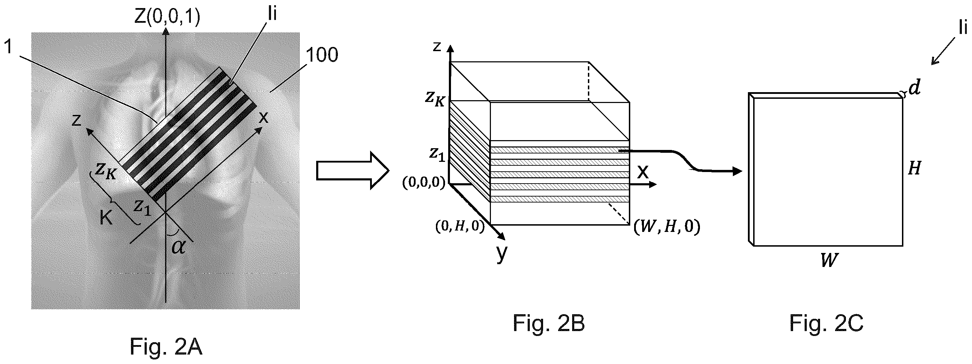

Figure 2A schematically illustrates a top view of a part of a breathing body 100 (a human chest in this example) comprising the heart 1.

A medical imaging device not illustrated in Figure 2A, comprises a snapshot module (which is an "image acquiring sensor") arranged for generating at least two snapshots $I_i$ (and in general N snapshots, N being a non-null and positive integer number, preferably equal or higher than two) of the heart 1 in a snapshot plane (which is an "imaging plane"), i.e. the plane of the snapshot imaged using the snapshot module.

The snapshot plane is perpendicular to the main plain of the patient (i.e. the plane XZ in Figure 1).

As visible in Figure 2A, the reference system xz of the snapshot planes is tilted by an angle $\alpha$ with regard to the external reference system X, Y, Z of Figure 1.

The angle $\alpha$ is between the local z-axis (of the snapshot planes reference system) and the global Z-axis (of the external reference system). In one embodiment, the angle $\alpha$ can be up to 40°. If it is more than that, the through-plane motion component lowers and the proposed method could become less effective.

The snapshot planes reference system xyz defines the image 3D space.

In the embodiment illustrated in Figures 2A and 2B, there are K imaging planes, K being a non-null and positive integer number, preferably equal or higher than two. At each $z_i$ ($z_i$ going from $z_1$ to $z_K$ in Figure 2A), there is a snapshot plane. At each $z_i$ position, N snapshots $I_i$ are acquired. The snapshot planes are transverse to the 3D portion to be reconstructed.

Each snapshot plane is a bi-dimensional element, having a width W and a height H, as visible e.g. in Figures 2B and 2C. The distance between two consecutive or adjacent snapshot planes is illustrated by the letter "d" in Figure 2C. In general, in the set of the K snapshot planes, the spacing between two consecutive or adjacent snapshot planes is uniform, and equal to d. In one embodiment, d is less than 10 mm, e.g. comprised between 4 mm and 6 mm.

Mathematically speaking, an image is a 2D matrix and does not have a thickness. But, when acquiring, the sensor can encode information in this "d" thickness into the image. Therefore, the image has thickness "d" which corresponds to the gap of "d" (which means no overlap between adjacent images).

Each snapshot plane is associated to a coordinate $z_i$, as visible in Figure 2A.

The snapshot module is arranged to move (or to be moved) relative to the 3D portion in a direction perpendicular to a set of K parallel snapshot planes (the direction z in Figure 2A), generating two images for each snapshot plane within the set of K parallel snapshot planes.

In other words, the snapshot module is arranged to generate at least two images for a snapshot plane at a $z_i$ position, and then move (or be moved) to the consecutive or adjacent snapshot plane at a $z_{i+1}$ position (wherein the distance between the snapshot plane at the $z_i$ position and the snapshot plane at the $z_{i+1}$ position is equal to d) and then generate at least two images for the snapshot plane at a $z_{i+1}$ position. Then, the snapshot module is arranged to move (or to be moved) to the consecutive or adjacent snapshot plane at a $z_{i+2}$ position (wherein the distance between the snapshot plane at the $z_{i+1}$ position and the snapshot plane at the $z_{i+2}$ position is still equal to d) and then generate at least two images for the snapshot plane at a $z_{i+2}$ position, and so on.

In one embodiment, a motion module of the medical imaging device is arranged for generating a relative motion between the snapshot module and the heart 1 of the breathing body 100 in a through-plane axis (e.g. the z axis) substantially perpendicular to the snapshot planes. In one embodiment, the motion module is arranged to move the snapshot module from a $z_i$ position to the consecutive or adjacent $z_{i+1}$ position.

In other words, the snapshot module is arranged for generating at least two snapshots of the 3D portion for each snapshot plane of K different snapshot planes, the K different snapshot planes being substantially parallel and perpendicular to the main plane of the patient, i.e. transverse to the 3D portion to be reconstructed.

In one preferred embodiment, the snapshot module is arranged for generating 24 to 32 snapshots of the heart 1 in each snapshot plane. This range is a good trade-off between the medical examination time and the final resolution of the reconstructed heart's volume. In fact, in one preferred embodiment, the 24 to 32 snapshots of the heart 1 are averaged, so as to reduce or suppress the noise. This renders the method for reconstructing the super-resolution volume of the 3D portion of the breathing body more robust. The averaging can be arithmetic, geometric, harmonic, quadratic and/or weighted, etc.

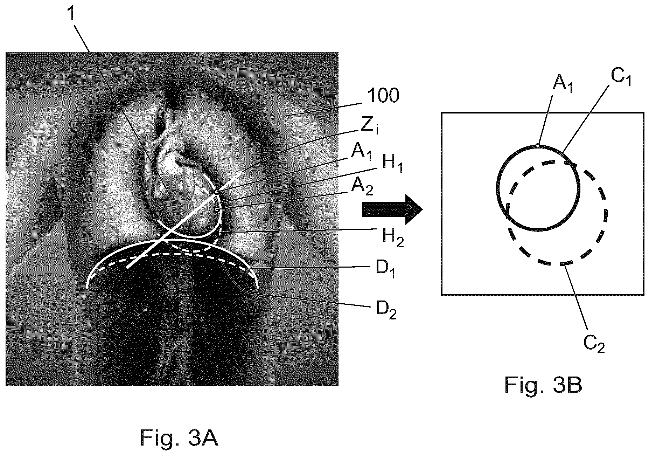

During the medical examen, the patient is freely breathing, as illustrated by the diaphragm's motion from a first position $D_1$ to a second position $D_2$ in Figure 3A, for a given snapshot plane defined by $z_i$. The heart moves as well from a

first position $H_1$ to a second position $H_2$.

Due the breathing, a given point of the heart belongs to a given snapshot plane when the heart 1 is in its first position $H_1$ (e.g. point's position $A_1$ in Figure 3A), but it doesn't belong anymore to this snapshot plane when the heart is in its second position $H_2$ (cf point's position $A_2$ in Figure 3A).

Moreover, when the heart 1 is in its second position $H_2$, the area of the heart's cross-section having the contour $C_2$ due to the snapshot plane is different from the area of the heart's cross-section having the contour $C_1$ due to the snapshot plane when the heart is in its first position $H_1$, as visible in Figure 3B. The two cross-sections have different sizes, since the heart is not a perfect cylinder. A shape's change is also possible.

Therefore, as illustrated in Figure 3B, there are two components of the heart's motion related to the breathing:

- an in-plane motion, i.e. a motion in a given snapshot plane, and
- a through-plane motion, i.e. a motion in a direction substantially perpendicular to the given snapshot plane.

In other words, the term "in-plane" indicates the plane of each image, and the term "through-plane" indicates a direction perpendicular to that plane.

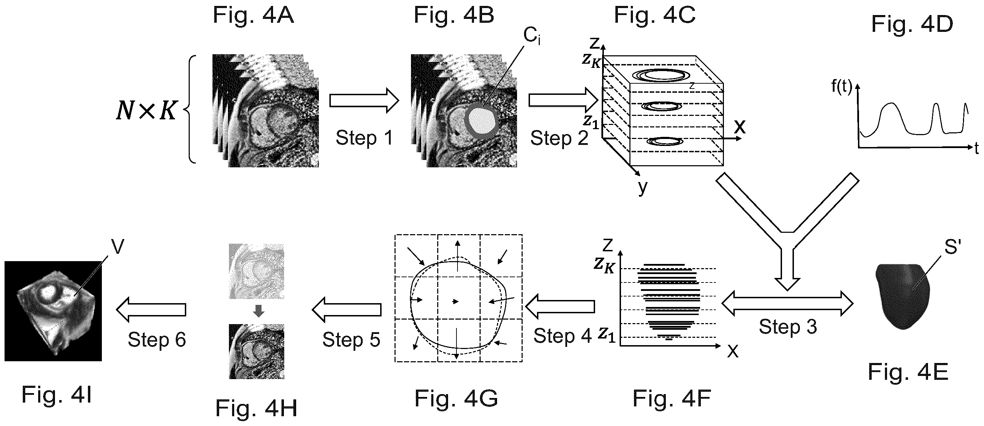

N snapshots (wherein preferably $N \geq 2$) at a given cross-section of the heart are then acquired. This procedure is repeated for K cross-sectional positions from $z_1$ to $z_K$ as indicated in the Figure 2A. During this acquisition, the heart 1 is moving as the patient is breathing. The system according to the invention allows to reconstruct a 3D image volume of the heart 1, an example of which is represented in Figure 4I, from the $N \times K$ acquired snapshots $I_i$, illustrated in Figure 4A. There are then N snapshots per each snapshot location $z_i$ of the K possible snapshot locations $z_i$.

In one embodiment, the snapshot planes are square or rectangular and have all the same dimensions. If the dimensions of a given snapshot plane are $H \times W$, the 3D image volume V of the heart 1 in the image 3D space will be :

$$V = D' \times H' \times W' \tag{1}$$

wherein

$$D' = S_z \times K$$

$$H' = S_y \times H$$

$$W' = S_x \times W \tag{2}$$

wherein

$S_x$, $S_y$, and $S_z$ are super-resolution factor, e.g. S being comprised in the range 2 to 8, e.g. S = 6
W is the snapshot first dimension (e.g. the width) in pixel along an axis of the snapshot plane, e.g. the x-axis
H is the snapshot second dimension (e.g. the height) in pixel along another axis of the snapshot plane, e.g. the y-axis, perpendicular to the x-axis D' is the snapshot third dimension (e.g. the depth) in pixel along an axis perpendicular to the snapshot plane, e.g. the z-axis.

In one embodiment, the system comprises a mask computing module (step 1 between Figures 4A and 4B), arranged to compute for the snapshots of each snapshot plane (at least) a mask, e.g. a binary mask, for the inner and/or outer contour of the 3D portion of each snapshot plane, preferably for both the inner and outer contours of the 3D portion of each snapshot plane. In one embodiment, a mask represents the segmentation of the 3D portion in each acquired (2D) snapshot, it is then a segmentation mask.

In one embodiment, as visible e.g. in Figure 4B, $N \times K$ masks, in particular $N \times K$ segmentation masks, are then generated.

The system comprises also a contour extraction module (step 2 between Figures 4B and 4C) arranged for detecting or extracting from the masks at least part of the contour $C_i$, and preferably all the (inner and/or outer) contour, of the heart for the snapshots of each snapshot plane, as illustrated in Figures 4B and 4C.

In one embodiment, the contour extraction module is also arranged to perform post processing on the masks, thereby generating cleaned masks. In one embodiment, the post processing comprises filtering out false positive regions from the masks, e.g. by using morphological operations.

In one embodiment, after the extraction of the masks, the contour extraction module is also arranged to place the contours back into their corresponding original (x,y,z)-locations in the image 3D space, along the through-plane axis z and in-plane axes x,y.

In one embodiment, an iterative 3D shift estimation module (step 3 between Figures 4C to 34F) is arranged for iteratively estimating a 3D shift of the extracted contours of Figure 4C.

In one embodiment, this estimation can also be based on a breathing pattern signal (on example of which is visible in Figure 4D).

In one embodiment, the breathing pattern is obtained from an external sensor attached to the patient, e.g. an external respiratory sensor.

In one embodiment, the breathing pattern is obtained from a motion analysis from the snapshots themselves.

The iterative 3D shift estimation module is arranged to compute this shift by using two iterative sub-steps: a forward step and a backward step.

In the forward step, the iterative 3D shift estimation module is arranged to use the extracted contours to estimate an approximate 3D shape S' of the 3D portion, visible e.g. in Figure 4E.

In the backward step, the iterative 3D shift estimation module is arranged to determine, for each extracted contour, the optimal 3D shift (dx, dy, dz) that minimizes the discrepancy between the extracted contour and the estimated 3D shape S'.

In one embodiment, the optimal fit is computed by using the mean closest distance metric between contour points and the 3D surface of the shape S'.

In one embodiment, the breathing pattern can be used as a regularizer when determining the 3D shift.

The forward and backward steps are iteratively repeated to refine the 3D shape and the 3D shift correction.

In one embodiment, a local deformation field module (step 4 between Figures 4F and 4G) is arranged to compute a local deformation field, i.e. to further refine the contours' alignment by applying local deformations to the contours (Figure 4G). This allows to take into account slight changes in the 3D portion shape.

In one embodiment, the local deformation field module is arranged to optimize the applied local deformation, e.g. such that the chamfer distance between the deformed contours and the estimated 3D shape is minimized.

In one embodiment, a colour normalization module (step 5 between Figures 4G and 4H) is arranged to perform a snapshot colour normalization, e.g. by normalizing intensity variations across snapshots to ensure consistent contrast and brightness in the final super-resolution volume.

In one embodiment, the colour normalization module is arranged to:

- estimate for each snapshot a white and black reference by using the segmentation masks previously computed by the mask computing module,
- use this reference to normalize each snapshot separately, ensuring uniform intensity levels across all images.

A super-resolution reconstruction module (step 6 between Figures 4H and 4I) is arranged for repositioning in the image 3D space all snapshots according to the computed 3D shift, for sampling the image 3D space with a super-resolution factors, and for computing voxel intensities in the sampled image 3D space by averaging voxel values from the (overlapping) snapshots, so as to reconstruct the super-resolution volume V of the 3D portion 1.

In one embodiment, the super-resolution reconstruction module is arranged also to apply the computed local deformation fields to their corresponding snapshots. In one embodiment, the local deformation field accounts for (slight) changes in the shape of the heart.

In one embodiment, the 3D shift accounts for both in-plane and through-plane motion.

In one embodiment, at least one module of the mentioned modules (mask computing module, contour extraction module, iterative 3D shift estimation module, local deformation field module, colour normalization module, super-resolution reconstruction module) is a machine learning-based module.

In this context a machine learning-based module is a module which needs to be trained in order to learn i.e. to progressively improve a performance on a specific task. In a preferred embodiment, the machine learning-based module is an artificial neural network, or neural network for short. It comprises a set of artificial neurons that are connected to each other to form a weighted directed graph. The neurons receive inputs from other neurons that feed into them, operate on these inputs, and transmit the results to the neurons they feed into. The edge weights denote the influence that a neuron has on those it is connected to and are computed by a process called learning which involves minimizing a loss function.

In one embodiment, the machine learning-based module is a neural network module.

In one embodiment, at least two of the mentioned modules, and preferably all the mentioned modules are implemented in a single computing unit.

In one embodiment, the medical imaging device comprises at least two of the mentioned modules, and preferably all the mentioned modules.

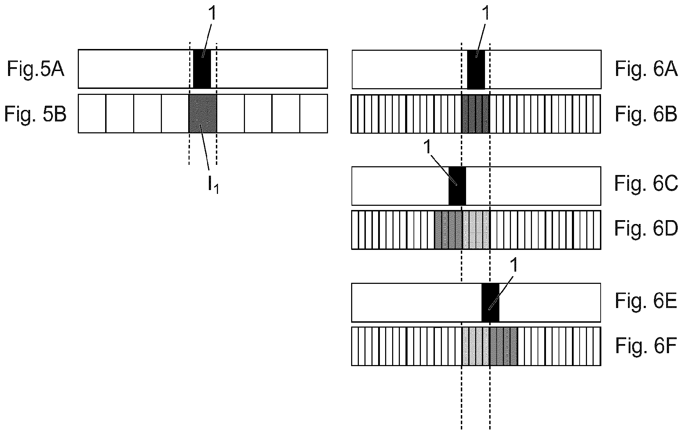

Figures 5A to 9C illustrate different embodiments that can be performed by the super-resolution reconstruction module, in particular for sampling snapshots in the image 3D space with a super-resolution factor. Reference can be made to a 1D object (instead of a 3D one) for explication purposes. Considerations made for a 1D object can be applied to each dimension of the three dimensions of a 3D object.

Figure 5A illustrates schematically a 1D object 1. Figure 5B schematically illustrates the captured image of the 1D object of Figure 5A, wherein each rectangle represents an image's pixel. Then, Figures 5A and 5B illustrate a single acquisition of an 1D object 1.

Figures 6A to 6F illustrate a multi acquisition of an 1D object 1. In figures 6B, 6D and 6F, each pixel has been divided in a number of sub-pixels, four in the illustrated example.

In particular, Figure 6A illustrates schematically a 1D object 1 in a first position. Figure 6B schematically illustrates the captured image of the 1D object of Figure 6A. Figure 6C illustrates schematically the 1D object 1 in a second position. Figure 6D schematically illustrates the captured image of the 1D object of Figure 6C. The 1D object is detected by two pixels. Figure 6E illustrates schematically the 1D object 1 in a third position. Figure 6F schematically illustrates the captured image of the 1D object of Figure 6E. The 1D object is detected by two pixels.

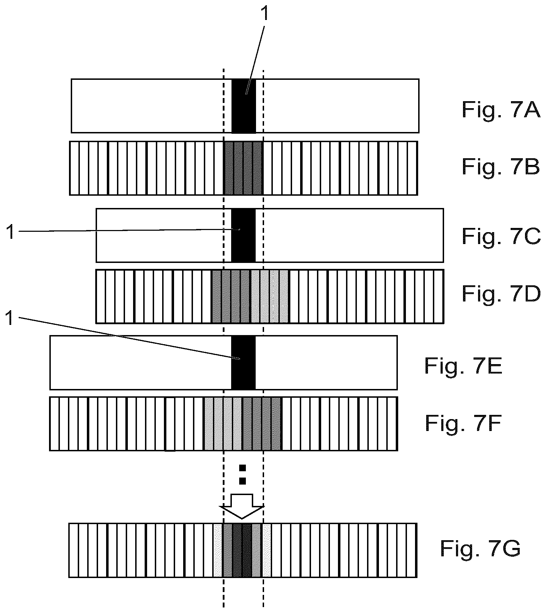

By aligning the acquired images of Figure 6B, 6D and 6F on the basis on the same position for the moving object 1, as illustrated in Figures 7A to 7F, and by considering the fact that in some cases the 1D object has detected by two pixels (and the corresponding sub-pixels), it is possible to reconstruct a super-resolution image, illustrated in Figure 7G, wherein the different greyscales correspond to a different probability for the object to be in a given sub-pixel.

The repositioning the acquired snapshots (in particular in all 3 axis with the computed dx,dy,dz shifts) corresponds to the alignment of Figures 7A to 7F and allows to obtain a super-resolution volume of the heart.

In one embodiment, the pixels of the acquired snapshots are subdivided in sub-pixels along the x,y, and z-axis. The number of the sub-divisions for each pixel is the super-resolution factor $S_x$, $S_y$, and $S_z$ defined in formula (2). This could be in the range from 2 to 8.

In one embodiment, the pixels (or voxels) of the acquired snapshots are subdivided also in sub-pixels (or sub-voxels) on the xy-plane as well, wherein the number of the sub-divisions for each pixel can be in the range from 2 to 4: in this case the principle of Figures 7A to 7F is applied along the three axes x, y, and z. In this case, when alignment happen, it happens in 3D. So, the contours get placed in its correct position not just along z-axis but also on xy-plane (snapshot plane) as well.

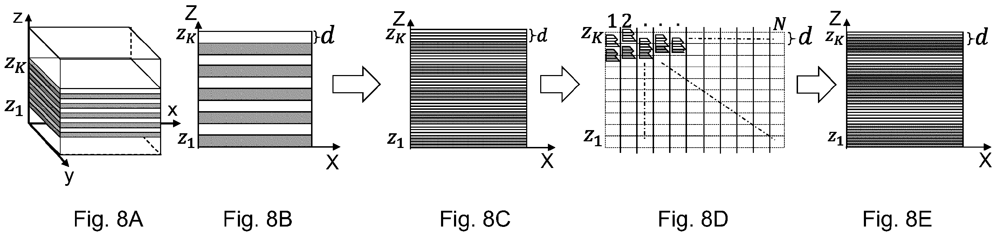

Figures 8A to 8E schematically illustrate some steps of the method according to one embodiment the invention.

Figure 8A illustrates the K snapshots in the 3D image space. Figure 8B illustrates the K snapshots along the z-axis.

Those K snapshots can then be re-sampled in a super-resolution grid, obtained by dividing the distance d between a location $z_i$ of a snapshot and the consecutive or adjacent location $z_{i+1}$ by an integer number, which is four in the illustrated example. Again, this number is the super-resolution factor S defined in formula (2).

The re-sampled snapshots can then be repositioned in the image 3D space according to the computed 3D shift (in Figure 8D, only the movement along the z direction is shown), so as to reconstruct the super-resolution volume of the object (a 2D section of which is shown in Figure 8E).

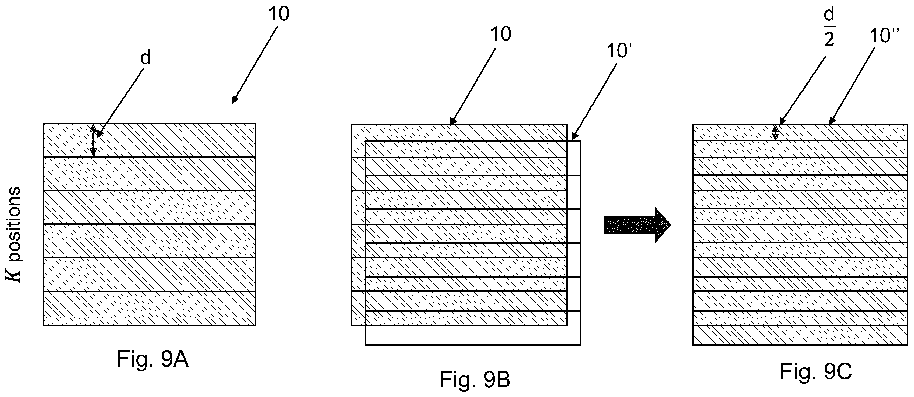

Figure 9A illustrates a schematic example of the group 10 of the K snapshot planes, i.e. of the snapshot planes for K locations. In one embodiment, the distance or the gap between the K locations, corresponding to a slice-thickness. In the example of Figure 9A, this distance is d.

Figures 9B and 9C illustrate an alternative embodiment, allowing to reduce the distance d, in order to improve the super-resolution obtained by the system according to the invention. In this embodiment, the medical imaging device, after having generated a first set 10 of snapshots at locations $z_i$ ($z_i$ going from $z_1$ to $z_K$), is arranged to perform a shift of the set of the snapshots in the image 3D space (in particular in the plane xz, and more in particular along the z axis), thereby obtaining a second set 10' of K snapshots.

The second set 10' of the K different snapshot is substantially parallel and shifted with regard to the previously generated set 10 of snapshots.

In one preferred embodiment, the shift is equal to d/2. The two shifted groups or sets 10, 10', each set comprising K snapshots, as visible in Figure 9B, are then partially superposed, as illustrated in Figure 9C, so as to allow an over-sampling of the heart. In the example illustrated in Figure 9C, thanks to this superposition, the distance or the gap between two consecutive or adjacent snapshots is equal to d/2.

In one embodiment, the medical imaging device is arranged to perform n shifts, wherein $n \geq 2$. However, the higher n, the longer the medical examination time. The applicant has found that n = 2 is a good trade-off between the improving of the super-resolution of the volume of the heart and the medical examination time.

**Reference signs used in the figures**

1                3D portion of a breathing body

10, 10', 10"    Groups of K snapshot planes

| 100 | Breathing body |
| --- | --- |
| 200 | Medical imaging device |
| $A_1$ | Point's first position |
| $A_2$ | Point's second position |
| $C_i$ | Contour of the cross-section of the hearth due to a snapshot plane |
| $D_1$ | Diaphragm's first position |
| $D_2$ | Diaphragm's second position |
| d | Distance |
| $H_1$ | Heart's first position |
| $H_2$ | Heart's second position |
| $I_i$ | Snapshot |
| K | Number of snapshot planes |
| S' | 3D shape |
| V | Volume |
| x | In-plane axis |
| $x_i$ | Displacement along the in-plane axis x |
| X, Y, Z | Global coordinate system of the medical imaging device |
| y | In-plane axis |
| z | Through-plane axis |
| $z_i$ | Displacement along the through -plane axis x |
| $\alpha$ | angle |

**Claims**

1. A free-breathing system for reconstructing a super-resolution volume (V) of a 3D portion (1) of a breathing body (100), the system comprising:

- a medical imaging device (200) comprising:

- a snapshot module arranged to move or to be moved relative to the 3D portion in a direction perpendicular to a set of K parallel snapshot planes, generating at least two snapshots for each plane within the set of K parallel planes transverse to the 3D portion, K being a non-null and positive integer number, while the body (100) is freely breathing, wherein each snapshot contains a 2D cross-section of the 3D portion

- a contour extraction module, arranged for extracting from the snapshot at least part of the contour ($C_i$) of the 2D cross-section for the snapshots ($I_i$) of each snapshot plane,
- an iterative 3D shift estimation module arranged for iteratively estimating a 3D shift in a 3D image space of the extracted contours, by using two iterative sub-steps:

- a forward step, in which the iterative 3D shift estimation module is arranged to use the extracted contours to estimate an approximate 3D shape of the 3D portion,
- a backward step, in which the iterative 3D shift estimation module is arranged to determine, for each extracted contour, the optimal 3D shift (dx, dy, dz) in the 3D image space that minimizes a discrepancy between the extracted contour and the estimated 3D shape,

- a super-resolution reconstruction module arranged for:

- repositioning in the image 3D space all snapshots according to the computed 3D shift,
- sampling the repositioned snapshots in the image 3D space with a super-resolution factor,
- computing voxel intensities in the sampled image 3D space by averaging voxel values from the snapshots, so as to reconstruct the super-resolution volume (V) of the 3D portion (1).

2. The free-breathing system of claim 1, comprising:

- a mask computing module, arranged to compute for the snapshots ($I_i$) of each snapshot plane a mask for an inner and/or outer contour of the 3D portion, the mask representing a segmentation of the 3D portion in each snapshot,

wherein the contour extraction module is arranged for extracting from the computed masks at least part of the contour ($C_i$).

3. The free-breathing system of claim 2, wherein the contour extraction module is also arranged to perform post processing on the masks, thereby generating cleaned masks.

4. The free-breathing system of one of claims 2 or 3, wherein after the extraction of the masks, the contour extraction module is also arranged to place the contours back into their corresponding original locations in the image 3D space.

5. The free-breathing system of one of claims 1 to 4, wherein a breathing pattern is used as a regularizer when determining the 3D shift.

6. The free-breathing system of claim 5, wherein the breathing pattern is obtained from an external sensor attached to the patient, or from a motion analysis from the snapshots themselves.

7. The free-breathing system of one of claims 1 to 6, comprising a local deformation field module arranged to compute a local deformation field, by applying local deformations to the contours.

8. The free-breathing system of one of claims 1 to 7, comprising a colour normalization module arranged to perform a snapshot colour normalization of the snapshots.

9. The free-breathing system of one of claims 1 to 8, wherein sampling the repositioned snapshots in the image 3D space with a super-resolution factor comprises dividing the distance d between a location ($z_i$) of a snapshot and the consecutive or adjacent location ($z_{i+1}$) by an integer number.

10. The free-breathing system of one of claims 1 to 9, wherein the snapshot module is arranged for generating 24 to 32 snapshots ($I_i$) of the 3D portion (1) in each snapshot plane ($P_i$).

11. The free-breathing system of one of claims 1 to 10, wherein the 3D portion (1) of the breathing body (100) is at least a portion of an organ, e.g. a portion of a heart.

12. The free-breathing system of one of claims 1 to 11, wherein at least one module of the modules is a machine learning-based module.

13. The free-breathing system of one of claims 1 to 12, wherein the 3D portion (1) of the breathing body (100) is at least a portion of an organ, e.g. a portion of a heart.

14. The free-breathing system of one of claims 1 to 13, wherein the medical imaging device is an MRI imaging device or a CT imaging device.

15. A free-breathing method for reconstructing a super-resolution volume of a 3D portion of a breathing body, comprising the steps of:

- moving a snapshot module of a medical imaging device arranged relative to the 3D portion in a direction perpendicular to a set of K parallel snapshot planes,
- generating at least two snapshots for each plane within the set of K parallel planes transverse to the 3D portion, K being a non-null and positive integer number, while the body is freely breathing,
- extracting by a contour extraction module from the snapshot at least part of the contour of the 3D portion for the snapshots of each snapshot plane,
- iteratively estimating a 3D shift in a 3D image space of the extracted contours by an iterative 3D shift estimation module, by using two iterative sub-steps:

  - a forward step, in which the iterative 3D shift estimation module is arranged to use the extracted contours to estimate an approximate 3D shape of the 3D portion,
  - a backward step, in which the iterative 3D shift estimation module is arranged to determine, for each extracted contour, the optimal 3D shift in the 3D image space that minimizes a discrepancy between the extracted contour and the estimated 3D shape,

- repositioning by a super-resolution reconstruction module in the image 3D space all snapshots according to the computed 3D shift,
- sampling by the super-resolution reconstruction module the repositioned snapshots in the image 3D space with a super-resolution factor,
- computing voxel intensities in the sampled image 3D space by the super-resolution reconstruction module, by averaging voxel values from the snapshots, so as to reconstruct the super-resolution volume of the 3D portion.

Fig. 1

Fig. 2A

Fig. 2B

Fig. 2C

Fig. 3A

Fig. 3B

Fig. 4A

Fig. 4B

Fig. 4C

Fig. 4D

Fig. 4E

Fig. 4F

Fig. 4G

Fig. 4H

Fig. 4I

Fig.5A

Fig. 5B

Fig. 6A

Fig. 6B

Fig. 6C

Fig. 6D

Fig. 6E

Fig. 6F

Fig. 7A

Fig. 7B

Fig. 7C

Fig. 7D

Fig. 7E

Fig. 7F

Fig. 7G

Fig. 8A          Fig. 8B          Fig. 8C          Fig. 8D          Fig. 8E

Fig. 9A

Fig. 9B

Fig. 9C

$K$ positions

| | Europäisches Patentamt European Patent Office Office européen des brevets | **EUROPEAN SEARCH REPORT** | **Application Number** EP 25 16 3520 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHUZHOU JIANG ET AL: "MRI of Moving Subjects Using Multislice Snapshot Images With Volume Reconstruction (SVR): Application to Fetal, Neonatal, and Adult Brain Studies", IEEE TRANSACTIONS ON MEDICAL IMAGING, IEEE, USA, vol. 26, no. 7, 1 July 2007 (2007-07-01), pages 967-980, XP011186645, ISSN: 0278-0062, DOI: 10.1109/TMI.2007.895456 * the whole document * | 1-15 | INV. G01R33/56 A61B6/03 G06T3/4053 |
| A | AMEROM JOSHUA F.P. ET AL: "Fetal whole-heart 4D imaging using motion-corrected multi-planar real-time MRI", MAGNETIC RESONANCE IN MEDICINE , 12 May 2019 (2019-05-12), XP093037418, US ISSN: 0740-3194, DOI: 10.1002/mrm.27798 Retrieved from the Internet: URL:https://onlinelibrary.wiley.com/doi/full-xml/10.1002/mrm.27798 * the whole document * | 1-15 | |
| A | EBNER MICHAEL ET AL: "An automated framework for localization, segmentation and super-resolution reconstruction of fetal brain MRI", NEUROIMAGE, ELSEVIER, AMSTERDAM, NL, vol. 206, 6 November 2019 (2019-11-06), XP086008137, ISSN: 1053-8119, DOI: 10.1016/J.NEUROIMAGE.2019.116324 [retrieved on 2019-11-06] * the whole document * | 1-15 | |

TECHNICAL FIELDS SEARCHED (IPC)

G01R
A61B
G06T

-/--

The present search report has been drawn up for all claims

| Place of search | Date of completion of the search | Examiner |
|---|---|---|
| Munich | 18 July 2025 | Durst, Markus |

Europäisches
Patentamt

European
Patent Office

Office européen
des brevets

**EUROPEAN SEARCH REPORT**

Application Number

EP 25 16 3520

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FERRANTE ENZO ET AL: "Slice-to-volume medical image registration: A survey", MEDICAL IMAGE ANALYSIS, OXFORD UNIVERSITY PRESS, OXOFRD, GB, 28 April 2017 (2017-04-28), XP085107048, ISSN: 1361-8415, DOI: 10.1016/J.MEDIA.2017.04.010 * the whole document * | 1-15 | |
| A | KERAUDREN K ET AL: "Automated fetal brain segmentation from 2D MRI slices for motion correction", NEUROIMAGE, ELSEVIER, AMSTERDAM, NL, vol. 101, 22 July 2014 (2014-07-22), pages 633-643, XP029054779, ISSN: 1053-8119, DOI: 10.1016/J.NEUROIMAGE.2014.07.023 * the whole document * | 1-15 | |
| A | Yezzi Anthony ET AL: "A Variational Framework for Joint Segmentation and Registration", Proceedings IEEE Workshop on Mathematical Methods in Biomedical Image Analysis (MMBIA 2001), 9 December 2001 (2001-12-09), XP093297388, Retrieved from the Internet: URL:https://ieeexplore.ieee.org/document/991698 * the whole document * | 1-15 | TECHNICAL FIELDS SEARCHED (IPC) |

The present search report has been drawn up for all claims

| Place of search | Date of completion of the search | Examiner |
|---|---|---|
| Munich | 18 July 2025 | Durst, Markus |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AMEROM JOSHUA et al.** *MAGENTIC RESONANCE IN MEDICINE*, 12 May 2019, ISSN 0740-319